# FASCICULE DE BREVET EUROPEEN

(11) **EP 1 265 983 B1**
(45) Date de publication et mention de la délivrance du brevet: **07.05.2008**
(21) Numéro de dépôt: 01917167.7
(22) Date de dépôt: 16.03.2001
(51) Int. Cl.: C12N 1/04, C12N 11/04, A23L 1/03, A23L 1/30, A61K 35/66

(54) **PROCEDE DE PRODUCTION DE PARTICULES CONTENANT DES MICROORGANISMES VIVANTS ENROBES.**
VERFAHREN ZUR HERSTELLUNG VON TEILCHEN, DIE LEBENDE, EINGEKAPSELTE MIKROORGANISMEN ENTHALTEN.
METHODS FOR PRODUCING PARTICLES CONTAINING COATED LIVING MICRO-ORGANISMS.

(30) Priorité: 16.03.2000 FR 0003409
(43) Date de publication de la demande: 18.12.2002
(73) Titulaire: Lallemand SAS, 31700 Blagnac (FR)
(72) Inventeur: DURAND, Henri, F-31520 Ramonville Saint-Agnes (FR); PANES, Jérôme, F-31400 Toulouse (FR)
(74) Mandataire: Peaucelle, Chantal
(86) Numéro de dépôt international: PCT/FR2001/000797
(87) Numéro de publication internationale: WO 2001/068808

(56) Documents cités:
- WO-A-92/12234
- DE-A- 3 738 599
- US-A- 4 888 171
- US-A- 5 507 871

## Description

La présente invention est relative à un procédé de production de particules contenant des microorganismes vivants déshydratés enrobés à l'aide d'une substance permettant d'augmenter la résistance desdits microorganismes aux stress physico-chimiques tels que la chaleur, l'humidité, l'acidité, ou la compression. La présente invention concerne également les applications de ces particules dans des compositions pharmaceutiques, diététiques ou alimentaires.

L'utilisation de microorganismes vivants déshydratés dans les applications pharmaceutiques, diététiques ou agro-alimentaires impose souvent que, lors de leur fabrication, de leur stockage ou de leur mise en oeuvre, ces microorganismes soient soumis à des stress physico-chimiques tels que par exemple des conditions de pression, de température, d'humidité ou d'acidité élevées. Ceci a pour effet d'affecter leur capacité à reprendre une activité normale après réhydratation, quand leur taux de survie n'est pas tout simplement extrêmement réduit par l'agressivité de tels traitements.

Diverses solutions ont été proposées, qui visent à recouvrir les microorganismes d'une substance protectrice.

Par exemple, le brevet WO 99/49846 enseigne l'enrobage de bactéries par des polymères, tels que les polyacrylamides, ou des co-polymères associés à des phospholipides. Cependant, de tels polymères ne sont pas autorisés dans les produits alimentaires ou pharmaceutiques.

Le brevet FR 96 06215 décrit l'enrobage de bactéries par des polymères ou des polysaccharides hydrosolubles, ce qui exclut toute stabilisation en milieu aqueux. En particulier, pour la conservation de produits contenant des microorganismes déshydratés, il est impératif de garantir un taux d'humidité très faible, afin qu'aucune reprise d'activité ne soit possible avant l'emploi dans le milieu voulu.

C'est pourquoi d'autres méthodes ont été proposées, utilisant à titre de matière protectrice, des substances comestibles et résistantes à l'humidité.

Selon la méthode décrite dans le document DE 3738599, des levures sont encapsulées dans des matières grasses, telles que des esters d'acides gras, dont la température de ramollissement est supérieure à 29°C. On retarde de la sorte de quelques heures le démarrage de la fermentation panaire dans des pâtons fiais ou décongelés, en mettant à profit le ramollissement progressif de la substance d'enrobage utilisée. De telles particules ne permettent donc pas d'assurer une protection mécanique contre la compression, ni une protection thermique efficace.

Le brevet WO 9212224 concerne des bactéries déshydratées encapsulées dans des acides gras, et plus précisément *Enterococcus faceium* encapsulée dans l'acide stéarique. Les capsules sont formées à partir d'une pâte composée de bactéries et d'acide gras intimement mélangés à raison de 50 à 90% en poids d'acide gras, et se présentent comme des microsphères de liant dans lesquelles les bactéries sont dispersées de manière aléatoire. La taille des particules obtenues varie entre 75 et 300 µm. Les particules obtenues sont utilisables comme probiotiques. De telles capsules ne comportent pas une couche de protection déposée régulièrement sur le microorganisme apportant une stabilité chimique et une résistance mécanique et thermique homogènes. Le procédé de fabrication mêlant le liant en fusion et la bactérie limite cette technique à l'enrobage des rares microorganismes survivant à un traitement à des températures élevées, la température de fusion de l'acide stéarique étant de 70±1°C. De plus, la taille des microsphères obtenues est comprise entre 75 et 300 µm, ce qui limite encore l'application du procédé à l'enrobage d'amas cellulaires initiaux de taille inférieure à ces valeurs (et pose en outre des problèmes de colmatage lors des manipulations).

Le brevet US 4 888 171 décrit un produit granulaire constitué d'un noyau, en sucre cristallisé par exemple, celui-ci étant enrobé d'une composition y adhérant, formée du mélange d'un liant de point de fusion compris entre 25 et 60°C et de cellules bactériennes séchées. Le produit obtenu a une structure stratifiée entourant le noyau. Ce produit est préparé par un procédé selon lequel le liant est vaporisé dans une chambre de granulation de manière concomitante avec l'alimentation en cellules bactériennes séchées, la température ambiante de la chambre étant comprise entre 25 et 55°C. Ainsi, pour un enrobant ayant un point de fusion supérieur à 60°C, la chambre de granulation devra être maintenue à une température supérieure à 55°C, à laquelle les microorganismes sont partiellement tués ou sont tellement endommagés qu'ils périssent ensuite. C'est la raison pour laquelle ce procédé est restreint à l'emploi de matières d'enrobage dont le point de fusion est inférieur à 60°C.

Toutes les techniques décrites précédemment comportent des inconvénients liés à l'impératif de concilier des procédés de fabrication agressifs et des entités biologiques fragiles, ce qui constitue un obstacle à l'obtention d'une protection efficace et durable de microorganismes vivants, non surmonté jusqu'à présent.

Ainsi, les solutions techniques proposées ne permettent pas à la fois:
- de préparer des particules protectrices de microorganismes vivants,
- et de préserver les microorganismes durant la fabrication desdites particules.
En effet, jusqu'à présent soit la matière enrobante a un point de fusion assez bas pour ne pas endommager les microorganismes durant la préparation des particules, mais elle n'est pas solide à température ambiante et de telles particules ne peuvent assurer une protection efficace et durable; soit des conditions élevées de température sont appliquées pour obtenir la fusion de la matière devant recouvrir les cellules, permettant d'obtenir des particules solides et résistantes à température ambiante, mais nuisant gravement à la viabilité des microorganismes.

II apparaît donc, que jusqu'à présent, il n'a pas été possible de produire, àpartir d'agglomérats de cellules de microorganismes vivants déshydratés préexistants, des particules enrobées présentant une résistance élevée aux stress physico-chimiques tels que la chaleur, l'humidité, l'acidité gastrique, ou la pression de manière efficace et durable, et utilisables dans les applications de la diététique, la pharmacie, l'agroalimentaire.

Les particules obtenues selon l'invention présentent l'avantage d'une bonne résistance aux contraintes mécaniques, d'une excellente stabilité chimique dans des conditions de stockage peu contraignantes et pour l'utilisation recherchée, alliées une viabilité parfaite, grâce à la mise au point d'un procédé simple et de portée générale rendant désormais possible l'emploi d'un grand nombre de substances pour l'enrobage et la protection des microorganismes vivants les plus variés.

Le principe du procédé de préparation mis en oeuvre dans la présente invention repose sur le refroidissement rapide des micro-gouttes de substance enrobante lors de leur injection dans une chambre de granulation, la chambre contenant les microorganismes à enrober étant maintenue à une température régulée compatible avec la survie des microorganismes. Le choix de la substance enrobante n'est alors plus tributaire de la compatibilité entre son point de fusion et la température tolérée par le microorganisme lors de la préparation, mais uniquement des critères recherchés pour le produit final.

Il est possible par exemple d'utiliser un enrobant dont le point de fusion est supérieur à la température du corps humain, de telle sorte que les particules ne fondent pas et restent intactes lors de l'ingestion afin de ne libérer le ou les microorganismes utiles qu'une fois dans l'estomac. Si en plus l'enrobant choisi est apte à résister, du moins partiellement, à l'attaque des sucs gastriques, il sera possible d'apporter la flore utile au-delà de la barrière gastrique.

Les propriétés de résistance mécanique élevée sont obtenues par la combinaison de deux facteurs essentiellement. D'une part, la substance d'enrobage est choisie pour être à l'état solide à la température de conservation, et d'autre part, les particules sont formées d'une seule couche de matière hydrophobe, recouvrant de façon homogène l'agglomérat de cellules séchées, ne présentant pas de cavités affleurant la surface de manière à ne pas faciliter la formation de fissures et les attaques chimiques, ni la rupture et l'écrasement.

Enfin, les particules objets de la présente invention sont particulièrement faciles d'emploi, car elles ont une granulométrie qui leur confère des propriétés d'écoulement tout à fait appréciables lors de la préparation et de la manipulation des particules, celles-ci gardant une fluidité comparable à celle d'un liquide et ne provoquant pas de colmatage.

Ainsi, la présente invention, par un choix judicieux de différents paramètres chimiques et physiques qui seront exposés ci-après, propose un procédé de production de particules dotées de propriétés originales contenant des microorganismes vivants de nature variée, permettant de concilier les diverses exigences techniques au niveau de leur fabrication, de leur conservation et de leur mise en oeuvre.

La solution au problème posé réside en la réalisation de particules constituées d'un agglomérat de microorganismes vivants déshydratés et d'une couche d'enrobage hydrophobe homogène.

La couche hydrophobe est composée principalement d'une substance choisie parmi les matières grasses, et en particulier parmi les acides gras ou les cires, purs ou en mélange entre eux. La caractéristique essentielle de la couche d'enrobage est son point de fusion qui peut être compris entre 20 et 100°C, de préférence entre 30 et 80°C. Les acides gras saturés sont préférés et en particulier l'acide stéarique. L'acide palmitique peut également être utilisé pour ses qualités techniques. Les cires animales ou végétales, telle que la cire de Carnauba, conviennent également.

A cette couche d'enrobage il est possible d'ajouter d'autres molécules d'additifs, tels que des anti-oxydants, des sucres ou des protéines, connus pour améliorer la stabilité des microorganismes dans les conditions de fabrication et/ou de conservation des microorganismes enrobés selon l'invention.

La couche d'enrobant recouvre les amas cellulaires de manière homogène, c'est-à-dire, que son épaisseur varie peu et surtout qu'elle a une structure uniforme et ne contient pas d'inclusions ni de cavités pouvant affecter la régularité de sa surface et par là même sa rigidité et sa cohésion. Elle est également homogène chimiquement.

Le choix de composés compatibles avec les utilisations prévues des particules selon l'invention est bien entendu recommandé. En particulier on choisira de préférence des matières répondant au critère de qualité alimentaire.

Les microorganismes destinés à être enrobés sont des cellules vivantes séchées par lyophilisation, atomisation, ou sur lit fluidisé, de telle sorte qu'ils soient revivifiables. Ces techniques sont bien connues de l'homme de l'art. Après broyage, on obtient des agglomérats de cellules, qui se présentent sous forme de poudre plus ou moins fine. Ces amas cellulaires peuvent être sensiblement sphériques, ovoïdes ou allongés, lisses ou rugueux, réguliers ou irréguliers. Leur taille est comprise entre quelques de microns et plusieurs millimètres.

Une grande variété de microorganismes, même les plus fragiles et sensibles aux conditions du milieu, sont susceptibles d'entrer dans la préparation de particules selon l'invention, dans la mesure où le procédé de fabrication des particules est peu agressif comparé aux techniques connues antérieurement. Citons les bactéries à usage alimentaire, diététique ou pharmaceutique telles que par exemple les lactobacilles, notamment *Lactobacillus casei, L. casei rhamnosus, L. acidophilus, L. bulgaricus, L. brevis, L. helveticus;* les bifidobactéries; les streptocoques, notamment *Streptococcus thermophilus, Streptococcus salivarius*; les lactocoques tel que *Lactococcus lactis*; les pediocoques notamment *Pediococcus acidilactici*; les entérocoques; les levures des genres Saccharomyces (en particulier *Saccharomyces cerevisiae, S. boulardii*), Kluyveromyces, etc. Le choix d'un ou d'un mélange de ces microorganismes sera déterminé avant tout par l'application finale à laquelle il est destiné, les conditions de fabrication relativement douces proposées par la présente invention ne constituant pas le critère limitant.

Les poudres sèches des microorganismes mentionnés ci-dessus, obtenues par lyophilisation, par atomisation ou par séchage en lit fluidisé, conviennent comme matériel de départ, cette liste n'étant pas limitative. Ainsi, les particules selon l'invention peuvent aussi contenir des microorganismes appartenant à d'autres espèces et présentant un intérêt pour d'autres domaines d'application, tels que la cosmétique, la protection de l'environnement, les procédés industriels nécessitant par exemple l'apport ciblé d'additifs, ou tout autre domaine.

Dans les particules selon l'invention, la proportion de matière enrobante par rapport à la quantité de microorganismes est comprise entre 10 et 99% en poids, avantageusement entre 30 et 80%. Si la couche d'enrobage est trop fine, la protection sera insuffisante; inversement, si la couche est trop épaisse, la libération des microorganismes sera plus longue. Ce paramètre sera donc réglé selon l'application finale prévue, en fonction de la rapidité de libération des microorganismes désirée.

La concentration en bactéries viables dans les particules enrobées est induite à partir de la proportion relative entre la substance enrobante et la poudre séchée mise en oeuvre. En effet, le nombre de microorganismes par gramme de poudre sèche étant connu avant enrobage, on peut calculer la concentration par particule après enrobage. Cette valeur théorique est comparée à la valeur réelle mesurée par les méthodes classiques, à titre de contrôle.

La concentration en bactéries viables est exprimée en UFC/g: Unité Formant Colonie par gramme de particules enrobés du microorganisme concerné. La méthode de mesure se fait par dénombrement microbiologique sur boîte de Pétri, après dilution appropriée, selon les techniques connues de l'homme de l'art.

Les particules finales ont une taille contrôlée, dépendant de la taille-des agglomérats de microorganismes de la poudre de départ et de l'épaisseur de l'enrobant déposé, avec un diamètre moyen pouvant varier entre 100 et 5000 µm, de préférence entre 300 et 2000 µm. Il est possible de faire varier la taille des agglomérats lyophilisés en fonction de l'application prévue, en utilisant toute technique à la disposition de l'homme du métier, par exemple par un broyage prolongé.

La présente invention se rapporte à un procédé de production de particules de microorganismes enrobés vivants déshydratés tels que décrits précédemment, consistant à injecter dans une enceinte de la matière hydrophobe en fusion, dans une masse de microorganismes brassés en permanence par rotation du disque faisant office de fond de l'enceinte, et balayés par un courant d'air sec de température fixée.

Ce procédé est caractérisé en ce qu'une substance hydrophobe est injectée à une température supérieure à sa température de fusion, ladite température d'injection étant comprise entre 30 et 120°C, dans une enceinte contenant lesdits microorganismes brassés par rotation du fond de ladite enceinte et balayés par un flux d'air à une température comprise entre 10 et 50°C telle que la température dans l'enceinte ne dépasse pas de plus de 5°C la température de viabilité desdits microorganismes.

Il a en effet été trouvé de façon surprenante qu'il était possible d'obtenir, par injection de produits hydrophobes en fusion dans une enceinte où les particules étaient soumises à une agitation rotative et à un balayage par un flux d'air, les paramètres étant judicieusement choisis, des particules enrobées présentant les caractéristiques désirées de stabilité physique et chimique.

Le procédé d'enrobage utilise un appareillage standard du commerce, appelé granulateur, disponible dans différentes tailles compatibles avec des volumes de production de quelques centaines de grammes à plusieurs centaines de kilogrammes par opération.

La figure 1 donne une représentation schématique du dispositif. En référence à ce dessin, le dispositif comprend une enceinte en acier inoxydable, dont le fond (2) est constitué d'un disque animé d'un mouvement rotatif par un moteur (3). Un flux d'air est injecté par l'espace (4) entre le fond (2) et le corps (1) de l'enceinte. L'air s'échappe de l'enceinte par un filtre (5) placé dans la partie supérieure de l'enceinte. La masse des microorganismes en poudre (6) est brassée par la rotation du disque (2). Une buse (7) permet l'injection, par l'intermédiaire d'une pompe (8) du produit enrobant maintenu à une température supérieure à son point de fusion dans un récipient thermostaté (9).

Dans la mise en oeuvre du procédé selon l'invention, les paramètres dont le choix est critique pour l'obtention d'un enrobage homogène sont la température du produit enrobant, la température de l'air balayant l'enceinte, la vitesse de rotation, le débit d'injection de l'enrobant, et les masses de produits mis en oeuvre. Les exemples 1 à 4 illustrent des modes de réalisation particuliers de l'invention, sans toutefois en limiter la portée.

La température de la substance d'enrobage placée dans le récipient thermostaté (9) est comprise entre 30 et 120°C, de préférence entre 60 et 120°C. En tout état de cause elle doit être supérieure au point de fusion de ladite substance, que celle-ci soit un produit pur ou un mélange.

La température de l'air balayant l'enceinte de granulation est comprise entre 10 et 50°C. Elle est strictement contrôlée, de manière à ce qu'au moment de l'injection de l'enrobant en fusion, l'élévation de température subie par les microorganismes ne dépasse pas quelques degrés, au maximum 5°C. Pour certains microorganismes supportant mal les températures supérieures à 40°C, la température de l'enceinte sera bien sûr réduite.

La vitesse de rotation et le débit d'injection de l'enrobant sont des paramètres interdépendants et liés aux masses de produits mis en oeuvre. La vitesse de rotation est généralement comprise entre 50 et 500 rpm (rotation par minute). L'injection de l'enrobant peut être effectuée à l'aide d'une ou de plusieurs buses réparties à la périphérie de l'enceinte.

L'ensemble des paramètres sont également ajustés en fonction de la forme des agglomérats de cellules de départ, et de la nature du produit enrobant.

Les avantages du procédé tel que décrit ci-dessus résident dans la possibilité d'enrober de manière non agressive des amas de microorganismes de forme et de taille variées, par une couche hydrophobe résistante, permettant une protection efficace et durable, ainsi que dans les nombreuses possibilités d'application offertes par de telles propriétés du fait de la grande diversité des organismes pouvant être enrobés de la sorte, notamment dans les domaines pharmaceutique, diététique ou alimentaire.

L'invention a également pour objet l'utilisation des particules décrites précédemment dans les domaines pharmaceutique, diététique ou agroalimentaire. En particulier, l'invention permet l'addition de microorganismes dans différents produits alimentaires comme les céréales, la confiserie, le lait en poudre, dans des comprimés, etc... tout en permettant une viabilité suffisante desdits microorganismes pendant la fabrication desdits produits et pendant la durée du stockage précédant la consommation. Elle permet également de protéger les microorganismes contre l'acidité gastrique pour une meilleure activité dans l'intestin. Du fait des propriétés originales des particules selon l'invention, de nombreuses autres utilisations peuvent également être envisagées.

Les exemples suivants illustrent de façon non limitative, des modes de réalisation de la présente invention, et les paramètres utilisés pour la production de différents types de particules.

### · Exemple 1 : Enrobage de Lactobacillus acidophilus par l'acide stéarique

600 g d'une poudre lyophilisée de bactéries *Lactobacillus acidophilus*, souche R052, déposée à la CNCM sous le N° I-1722, commercialisée par l'Institut Rosell, 8480, boulevard Saint-Laurent, Montréal, Canada, sont introduits dans un granulateur à flux d'air de marque Glatt, modèle GPCG1, en configuration « rotor », ayant une capacité de 3 litres. 900 g d'acide stéarique (Fluka, référence 85683, point de fusion pF = 69-71°C) sont introduits dans le récipient thermostaté (9).

L'opération d'enrobage est conduite selon les paramètres suivants :
- vitesse de rotor: 300 rpm
- vitesse de l'air dans l'enceinte: 3 à 4 m/s soit ouverture du clapet d'entrée a 40%
- pression de pulvérisation de l'enrobant: 2 bars
- débit d'enrobant: 40 g/minute
- température de l'air de pulvérisation: 120°C
- température de l'acide stéarique: 100°C
- température de l'air d'entrée: 30°C
- température du produit: 32-35°C.

A la fin de l'opération, le granulateur est vidé, et les particules sont recueillies et stockées dans sachets hermétiques.

Les particules ainsi obtenues possèdent les caractéristiques suivantes:
- Le diamètre moyen des particules est de 400 µm, avec 92% compris entre 100 et 600 µm.
- La teneur en enrobant est de 60%.
- La concentration en bactéries viables de la poudre de départ est de 3,2.10¹¹ UFC/g (Unités Formant Colonies par gramme), celle des particules enrobées de 1,2.10¹¹ UFC/g de poudre introduite initialement.

### Exemple 2 : Enrobage de Lactobacillus casei par un mélange d'acide gras

600 g d'une poudre lyophilisée de *Lactobacillus casei*, souche EQ 85 (déposée a la CNCM sous le N° MA 64/4U), commercialisée par Lallemand SA, 15130 Saint Simon, sont introduits dans te même granulateur que celui utilisé dans l'exemple 1 et l'enrobant est placé dans le récipient thermostaté. Le produit enrobant est un mélange acide · stéarique / acide palmitique a parts égales, dont le point de fusion est pF = 55 °C, commercialisé par Exaflor (47, ailée de Chanteraine, 91190 Gif sur Yvette, France), sous la dénomination Stéarine^{™} 50/50.

Les paramètres utilisés sont les mêmes que ceux mentionnés dans l'exemple 1, à l'exception de :
- la température de l'enrobant : 80°C
- la température de l'air d'entrée : 25°C
- la température du produit : 28-32°C

Les particules obtenues présentent les caractéristiques suivantes:
- Elles ont un diamètre moyen de 750 µm, avec 90% compris entre 100 et 1000 µm.
- La teneur en acide gras est de 60%.
- La concentration en bactéries viables de la poudre de départ est de 4,8.10¹¹ UFC/g, celle des particules enrobées de 1,6.10¹¹ UFC/g.

### Exemple 3 : Enrobage de Saccharomyces cerevisiae par une cire végétale

750 g d'une préparation de levure sèche *Saccharomyces cerevisiae*, souche déposée à la CNCM sous le N° I 1079, commercialisée sous la marque Levucell SB, par Lallemand Sarl, 15130, Saint SIMON, sont introduits dans le granulateur A flux d'air utilisé à l'exemple 1, et enrobés avec 750 g d'une cire végétale, la cire de Carnauba, dont le point de fusion est pF = 83-88°C (commercialisée par Exaflor, Gif sur Yvette, France).

Les paramètres sont identiques à ceux de l'exemple 1, sauf :
- la température de la cire: 120°C
- la température de l'air d'entrée: 40°C
- la température du produit: 45-48°C

Les particules obtenues présentent les caractéristiques suivantes:
- Elles ont un diamètre moyen de 1200 µm, avec 90% compris entre 500 et 2500 µm.
- La teneur en acide gras est de 50%.
- La concentration en cellules viables de la poudre de départ est de 3.10¹⁰ UFC/g, celle des particules enrobées de 1,45.10¹⁰ UFC/g de poudre de départ.

### - Exemple 4 : Enrobage de Pediococcus acidilactici dans un mélange d'acides gras

80 kg de poudre lyophilisée de *Pediococcus acidilactici*, souche déposée à la CNCM sous le N° MA 18/5M, commercialisée par Lallemand SA, 15130, Saint SIMON, sous la marque Bactocell, sont introduits dans un granulateur GLATT^{(™)}, modèle CRG200, de 450 litres de capacité, équipé de 2 buses d'injection. Les cellules lyophilisées sont enrobés avec 160 kg de Stéarine 50/50^{(™)} (Exaflor, Gif sur Yvette, France).

Les paramètres de préparation des particules sont :
- vitesse du rotor: 120rpm
- débit d'air: 1500 à 2000 m³ / h
- pression de pulvérisation: 5 bars
- débit de l'acide gras: 800 g/mn (400 g/mn par buse, sur 2 buses)
- température de l'enrobant: 80°C
- température de l'air de pulvérisation: 120°C
- température de l'air à l'entrée : 25°C
- température du produit à 30°C ± 2°C

Les particules obtenues présentent les caractéristiques suivantes:
- Le diamètre moyen est de 400 µm, avec 87% compris entre 100et 600 µm.
- La teneur en enrobant est de 75%.
- La concentration en bactéries viables de la poudre de départ est de 3.10¹¹ UFC/g, celle des particules enrobées de 7,5.10¹⁰ UFC/g.

### Exemple 5: Stabilité thermique

La viabilité des bactéries *Lactobacillus acidophilus* dans la poudre lyophilisée utilisée dans l'exemple 1 d'une part, et dans les particules enrobées obtenues selon ce même exemple 1 d'autre part, a été étudiée dans les conditions suivantes:

Des échantillons de 10 grammes de chaque préparation sont introduits dans des tubes hermétiques, maintenus dans un bain-marie à 50°C. Un tube de chaque préparation est retiré du bain-marie après 1 heure, 4 heures, 7 heures et 24 heures, et la concentration en bactéries viables est immédiatement déterminée.

Les résultats sont consignés dans le tableau 1 ci-après. Les concentrations sont exprimées en UFC/g et en pourcentage de la concentration de chaque échantillon à T = 0 ) :

**TABLEAU 1**

| Temps | 0 | 1 mois | 2 mois | 3 mois | 4 mois |
|---|---|---|---|---|---|
| Poudre lyophilisée | 3,1.10⁹ | 1,3.10⁹ | 1,2.10⁹ | 9,9.10⁸ | 4,8.10⁸ |
| | (100%) | (42%) | (38%) | (32%) | (15%) |
| Particules enrobées | 7,5.10⁸ | 6,4.10⁸ | 5,5.10⁸ | 5,3.10⁸ | 5,2.10⁸ |
| | (100%) | (85%) | (73%) | (71%) | (69%) |

La stabilité à 50°C des bactéries sous forme de particules enrobées selon la présente invention est nettement améliorée par rapport à celle des bactéries sous forme de poudre lyophilisée.

### Exemple 6: Stabilité dans le lait en poudre

La viabilité des bactéries *Pediococcus acidilactici* dans la poudre lyophilisée utilisée dans l'exemple 4 d'une part, et dans les particules enrobées obtenues selon ce même exemple 4 d'autre part, a été étudiée dans les conditions suivantes:

Les préparations de bactéries sont mélangées à de la poudre de lait (marque Régilait) à raison de 1% en poids de préparation bactérienne pour 99% de poudre de lait. Les mélanges sont répartis en échantillons de 100 grammes dans des sachets en polyéthylène soudés. Les sachets sont maintenus à 30°C dans une étuve. Chaque mois, un sachet de chaque mélange est analysé pour la teneur en bactéries viables.

Les résultats sont consignés dans le tableau 2 ci-après. Les concentrations sont exprimées en UFC/g et en pourcentage de la concentration de chaque échantillon à T = 0.

**TABLEAU 2**

| Temps | 0 | 1 h | 4 h | 7 h | 24 h |
|---|---|---|---|---|---|
| Poudre lyophilisée | 3,1.10¹¹ | 2,9.10¹¹ | 1,1.10¹¹ | 9.10¹⁰ | 2,5.10⁹ |
| | (100%) | (93%) | (35%) | (29%) | (8%) |
| Particules enrobées | 1,2.10¹¹ | 1,2.10¹¹ | 1,2.10¹¹ | 1,1.10¹¹ | 9.10¹⁰ |
| | (100%) | (100%) | (100%) | (92%) | (69%) |

En présence de lait en poudre à 30°C, la stabilité des bactéries sous forme de particules enrobées selon la présente invention est nettement améliorée par rapport à celle des bactéries sous forme de poudre lyophilisée.

### Exemple 7 : Stabilité gastrique

La stabilité des bactéries *Lactobacillus acidophilus* dans des conditions simulant le passage dans l'estomac a été étudiée selon le protocole suivant:

Deux échantillons sont préparés, l'un à partir de la poudre lyophilisée telle qu'utilisée dans l'exemple 1 et l'autre à partir des particules enrobées selon ce même exemple 1. Pour chaque échantillon, 1 gramme de poudre lyophilisée ou de particules enrobées est introduit dans un flacon contenant 100ml d'une solution d'acide chlorhydrique 0,1N (pH 1,2). Le flacon est placé sous agitation dans un bain-marie à 37°C pendant 1 heure. La suspension est ensuite centrifugée, et le culot est repris dans 100 ml de tampon phosphate à pH 7,0. La concentration résiduelle en bactéries viables est alors déterminée, et comparée à celle d'un témoin où l'acide chlorhydrique est remplacé par une solution tamponnée à pH 7,0.

Dans ces conditions, les bactéries *Lactobacillus acidophilus* sous forme de poudre lyophilisée sont pratiquement entièrement détruites (survie inférieure à 0,01%), alors que les mêmes bactéries contenues dans les particules enrobées présentent un taux de survie de 15%.

La stabilité des bactéries *Lactobacillus casei* a été étudiée selon le même protocole:

Les bactéries *Lactobacillus casei* contenues dans la poudre lyophilisée telle qu'utilisée dans l'exemple 2 sont pratiquement entièrement détruites par le test gastrique (survie inférieure à 0,01 %), alors que les mêmes bactéries contenues dans les particules enrobées selon ce même exemple 2 présentent un taux de survie à ce même test de 25%.

### Exemple 8 : Stabilité à la compression

Des comprimés à base des microorganismes *Lactobacillus acidophilus, Lactobacillus casei, Saccharomyces cerevisiae* et *Pediococcus acidilactici,* séchés par lyophilisation, tels que décrits dans les exemples 1, 2, 3 et 4 respectivement, sont préparés selon le protocole suivante:

Pour chaque échantillon microbien, la poudre lyophilisée est mélangée à raison de 5% en poids dans un d'excipient composé de la manière suivante:
- 49% de Sorbitol (référence Neosorb 60w, UPSA),
- 49% de lactose (référence Fast flow, Seppic)
- et 2% de stéarate de magnésium (UPSA).

Chaque mélange est introduit successivement dans un appareil à comprimer de type alternatif, modèle EKOD commercialisé par Korsch. Les forces de compression exercées sont de 17500 N sur le piston supérieur et de 16400 N sur le piston inférieur.

Les concentrations en cellules viables sont déterminées dans chaque mélange avant compression, puis dans les comprimés. Les résultats, exprimés en pourcentage de survie après compression, sont consignés dans le tableau 3 suivant :

**TABLEAU 3**

| Espèce | *Lactobacillus acidophilus* | *Lactobacillus casei* | *Saccharomyces cerevisiae* | *Pediococcus acidilactici* |
|---|---|---|---|---|
| Poudre non enrobée | 44% | 3% | 2% | 48% |
| Particules enrobées | 98% | 10% | 16% | 100% |

La survie après compression des bactéries ou levures contenues dans les particules enrobées est nettement améliorée par rapport aux poudres non enrobées correspondantes.

## Revendications

1. Procédé d'enrobage de microorganismes vivants déshydratés par une couche hydrophobe homogène comprenant une substance choisie parmi les acides gras, les cires ou un mélange de ceux-ci ***caractérisé en ce que*** ladite substance hydrophobe est injectée à une température supérieure à sa température de fusion, ladite température d'injection étant comprise entre 30 et 120°C, dans une enceinte contenant lesdits microorganismes brassés par rotation du fond de ladite enceinte et balayés par un flux d'air à une température comprise entre 10 et 50°C telle que la température dans l'enceinte ne dépasse pas de plus de 5°C la température de viabilité desdits microorganismes.

2. Procédé d'enrobage de microorganismes vivants déshydratés selon la revendication 1, ***caractérisé en ce que*** la température d'injection de ladite substance hydrophobe est comprise entre 60 et 120°C.

3. Procédé d'enrobage de microorganismes vivants déshydratés la revendication précédente, ***caractérisé en ce que*** ladite substance hydrophobe d'enrobage a un point de fusion compris entre 20 et 100°C.

4. Procédé d'enrobage selon la revendication précédente ***caractérisé en ce que*** ladite substance hydrophobe d'enrobage a un point de fusion compris entre 30 et 80°C.

5. Procédé d'enrobage selon l'une des revendications précédentes, ***caractérisé en ce que*** lesdits microorganismes vivants déshydratés sont choisis parmi les lactobacilles, les bifidobactéries, les streptocoques, les entérocoques, les pédiocoques, les levures, ou un mélange de ceux-ci.

6. Procédé d'enrobage selon l'une des revendications précédentes, ***caractérisé en ce que*** la proportion de ladite substance enrobante injectée est comprise entre 10 et 99%, en poids de particules finales.

7. Procédé d'enrobage selon la revendication précédente, ***caractérisé en ce que*** la proportion de ladite substance enrobante injectée est comprise entre 30 et 80% en poids de particules finales.

8. Procédé d'enrobage de microorganismes vivants déshydratés selon l'une quelconque des revendications précédentes ***caractérisé en ce que*** la vitesse de rotation du fond de ladite enceinte est comprise entre 50 et 500 RPM.

9. Procédé d'enrobage de microorganismes vivants déshydratés selon l'une des revendications précédentes ***caractérisé en ce que*** le diamètre moyen des particules obtenues est compris entre 100 et 5000 µm.

10. Application d'un procédé selon l'une des revendications 1 à 9 à la préparation de particules destinées à la réalisation de compositions pharmaceutiques, diététiques ou alimentaires.

## Claims

1. Method of coating dehydrated living microorganisms by a homogeneous hydrophobic layer comprising a substance chosen from fatty acids, waxes or a mixture of the latter **characterized in that** said hydrophobic substance is injected at a temperature higher than its melting point, said injection temperature being comprised between 30 and 120°C, into a chamber containing said microorganisms which are stirred by rotation of the base of said chamber and swept by a flow of air at a temperature comprised between 10 and 50°C, such that the temperature in the chamber does not exceed by more than 5°C the viability temperature of said microorganisms.

2. Method of coating dehydrated living microorganisms according to claim 1, **characterized in that** the temperature of injection of said hydrophobic substance is comprised between 60 and 120°C.

3. Method of coating dehydrated living microorganisms according to the previous claim, **characterized in that** said hydrophobic coating substance has a melting point comprised between 20 and 100°C.

4. Coating method according to the previous claim, **characterized in that** said hydrophobic coating substance has a melting point comprised between 30 and 80°C.

5. Coating method according to one of the previous claims, **characterized in that** said dehydrated living microorganisms are chosen from lactobacilli, bifidobacteria, streptococci, enterococci, pediococci, yeasts, or a mixture of the latter.

6. Coating method according to one of the previous claims, **characterized in that** the proportion of said injected coating substance is comprised between 10 and 99%, by weight of the final particles.

7. Coating method according to the previous claim, **characterized in that** the proportion of said injected coating substance is comprised between 30 and 80% by weight of the final particles.

8. Method of coating dehydrated living microorganisms according to one of the previous claims, **characterized in that** the speed of rotation of the base of said chamber is comprised between 50 and 500 rpm.

9. Method of coating dehydrated living microorganisms according to one of the previous claims, **characterized in that** the average diameter of the particles obtained is comprised between 100 and 5000 µm.

10. Application of a method according to one of claims 1 to 9 to the preparation of particles intended for the production of pharmaceutical, dietetic or dietary compositions.

## Patentansprüche

1. Verfahren zum Umhüllen von dehydratisierten lebenden Mikroorganismen mit einer homogenen hydrophoben Schicht, die eine Substanz enthält, die aus Fettsäuren, Wachsen oder einem Gemisch derselben ausgewählt ist, **dadurch gekennzeichnet, dass** die hydrophobe Substanz bei einer Temperatur oberhalb ihrer Schmelztemperatur, bei einer Injektionstemperatur von 30 bis 120 °C, in einen Behälter injiziert wird, der die Mikroorganismen enthält, die durch Rotation des Bodens des Behälters umgerührt und von einem Luftstrom bei einer Temperatur von 10 bis 50 °C durchströmt werden, so dass die Temperatur in dem Behälter die Temperatur, bei der die Mikroorganismen lebensfähig sind, um nicht mehr als 5 °C übersteigt.

2. Verfahren zum Umhüllen von dehydratisierten lebenden Mikroorganismen gemäß Anspruch 1, **dadurch gekennzeichnet, dass** die Injektionstemperatur der hydrophoben Substanz 60 bis 120 °C beträgt.

3. Verfahren zum Umhüllen von dehydratisierten lebenden Mikroorganismen gemäß dem vorstehenden Anspruch, **dadurch gekennzeichnet, dass** die hydrophobe Umhüllungssubstanz einen Schmelzpunkt von 20 bis 100 °C hat.

4. Verfahren zum Umhüllen gemäß dem vorstehenden Anspruch, **dadurch gekennzeichnet, dass** die hydrophobe Umhüllungssubstanz einen Schmelzpunkt von 30 bis 80 °C hat.

5. Verfahren zum Umhüllen gemäß einem der vorstehenden Ansprüche, **dadurch gekennzeichnet, dass** die dehydratisierten lebenden Mikroorganismen aus Lactobacillen, Bifidobakterien, Streptokokken, Enterokokken, Pediokokken, Hefen oder einem Gemisch derselben ausgewählt sind.

6. Verfahren zum Umhüllen gemäß einem der vorstehenden Ansprüche, **dadurch gekennzeichnet, dass** der Anteil der injizierten umhüllenden Substanz 10 bis 99 Gew.-% beträgt, bezogen auf das Gewicht der fertigen Teilchen.

7. Verfahren zum Umhüllen gemäß einem der vorstehenden Ansprüche, **dadurch gekennzeichnet, dass** der Anteil der injizierten umhüllenden Substanz 30 bis 80 Gew.-% beträgt, bezogen auf das Gewicht der fertigen Teilchen.

8. Verfahren zum Umhüllen von dehydratisierten lebenden Mikroorganismen gemäß einem der vorstehenden Ansprüche, **dadurch gekennzeichnet, dass** die Rotationsgeschwindigkeit des Bodens des Behälters 50 bis 500 U/min beträgt.

9. Verfahren zum Umhüllen von dehydratisierten lebenden Mikroorganismen gemäß einem der vorstehenden Ansprüche, **dadurch gekennzeichnet, dass** der mittlere Durchmesser der erhaltenen Teilchen 100 bis 5000 µm beträgt.

10. Anwendung eines Verfahrens gemäß einem der Ansprüche 1 bis 9 auf die Herstellung von Teilchen zur Herstellung von pharmazeutischen, diätetischen oder Nahrungszusammensetzungen.
